(19) Europäisches Patentamt
European Patent Office
Office européen des brevets

(11) Publication number: **0 410 148 B1**

(12) **EUROPEAN PATENT SPECIFICATION**

(45) Date of publication of patent specification: **06.04.94**

(51) Int. Cl.[5]: **C07D 471/14**, A61K 31/55, //(C07D471/14,243:00,221:00, 221:00)

(21) Application number: **90112072.5**

(22) Date of filing: **26.06.90**

(54) **Novel 5,11-dihydro-6H-dipyrido[3,2-b:2',3'-e][1,4]diazepin-6-ones and thiones and their use in the prevention or treatment of AIDS.**

(30) Priority: **28.06.89 US 372974**

(43) Date of publication of application:
**30.01.91 Bulletin 91/05**

(45) Publication of the grant of the patent:
**06.04.94 Bulletin 94/14**

(84) Designated Contracting States:
**AT BE CH DE DK ES FR GB GR IT LI LU NL SE**

(56) References cited:
**EP-A- 0 066 774**
**EP-A- 0 312 895**
**DE-A- 1 916 011**
**DE-B- 1 620 572**

(73) Proprietor: **Boehringer Ingelheim Pharmaceuticals Inc.**
**90 East Ridge**
**P.O. Box 368**
**Ridgefield Connecticut 06877(US)**

Proprietor: **Dr. Karl Thomae GmbH**
**D-88397 Biberach(DE)**

(72) Inventor: **Hargrave, Karl D. Dr.**
**4 Edna Drive**
**Brookfield Conn. 06805(US)**
Inventor: **Schmidt, Günther Dr.**
**-**
**Deceased(DE)**
Inventor: **Engel, Wolfhard Dr.**
**Mozartstrasse 13**
**W-7950 Biberach 1(DE)**
Inventor: **Trummlitz, Günter Dr.**
**Buchenweg 27**
**W-7951 Warthausen(DE)**
Inventor: **Eberlein, Wolfgang Dr.**
**Obere Au 6**
**W-7950 Biberach 1(DE)**

(74) Representative: **Laudien, Dieter, Dr. et al**
**Boehringer Ingelheim GmbH**
**Abteilung Patente**
**D-55216 Ingelheim (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

This invention relates to novel 5,11-dihydro-6H-dipyrido[3,2-b:2′,3′-e][1,4]diazepin-6-ones and -thiones and the use of these compounds to combat HIV infection.

The human disease, Acquired Immune Deficiency Syndrome (AIDS), is considered to be caused by the Human Immunodeficiency Virus (HIV), particularly the strain known as HIV-1.

Like other viruses, HIV-1 cannot replicate without utilizing the biosynthetic apparatus of the host cell it infects. The virus causes the biosynthetic apparatus to produce the proteins needed for viral progency. The viral proteins are coded for by the genetic material contained within the infecting virus particle, or virion. In a retrovirus, such as HIV, the stable genetic material normally carried by the virus is RNA, compared to the host cell's DNA genome. The viral RNA must be converted into DNA and integrated into the host cell's genome, so that the host cell may produce the required viral proteins.

The conversion of the RNA to DNA is accomplished by the enzyme reverse transcriptase (RT), which is present within the infecting virion with the RNA. Reverse transcriptase has three identified enzymatic functions - it acts as an RNA-dependent DNA polymerase, as a ribonuclease, and as a DNA-dependent DNA polymerase. As an RNA-dependent DNA polymerase, RT produces a single-stranded DNA copy of the viral RNA. Next, as a ribonuclease, RT releases the newly-formed DNA produced from the original viral RNA and then destroys the original RNA. Finally, acting as a DNA-dependent DNA polymerase, RT makes a second, complementary DNA strand, using the first DNA strand as a template. The two DNA strands form a double-stranded DNA molecule, which is integrated into the host cell's genome by another enzyme called an integrase.

The genetic information of the host cell is stably maintained as double-stranded DNA. In normal protein synthesis, a selected section of the DNA genome is first transcribed into single stranded messenger RNA which is subsequently translated to produce the encoded proteins. There is no reverse transcription involved in the normal metabolism of the host cell and an enzyme displaying a reverse transcriptase activity is not present.

Hence compounds which inhibit the enzymatic functions of HIV reverse transcriptase (especially HIV-1 reverse transcriptase) will inhibit replication of HIV in infected cells without affecting the host cell. Such compounds are useful in the prevention or treatment of HIV infection in human subjects.

Viewed from one aspect, the invention provides compounds of Formula I

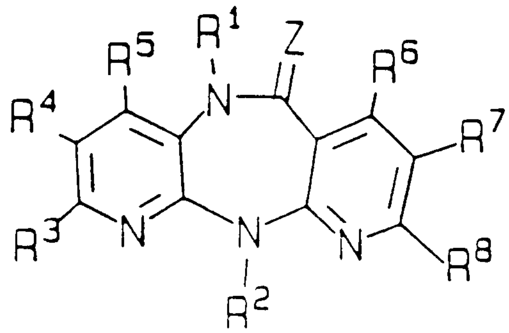

(I)

(wherein,

Z is oxygen or sulphur;

$R^1$ is hydrogen, $C_{1-5}$alkyl optionally substituted by fluorine, trihalomethyl, $C_{3-5}$alkenyl or alkynyl, 2-halopropen-1-yl, arylmethyl (wherein the aryl moiety is phenyl, thienyl or furanyl and is optionally substituted by methyl, methoxy or halogen), $C_{2-3}$ alkanoyl or $C_{2-4}$alkoxyalkyl or alkylthioalkyl;

$R^2$ is hydrogen, $C_{1-5}$ alkyl optionally substituted by fluorine, $C_{2-5}$alkenyl or alkynyl, $C_{2-4}$alkoxyalkyl or alkylthioalkyl, $C_{2-4}$alkanoyl, $C_{2-5}$hydroxyalkyl, arylmethyl (wherein the aryl moiety is phenyl, thienyl or furanyl, and is optionally substituted by $C_{1-3}$ alkyl or alkoxy, hydroxyl or halogen), phenyl optionally substituted by $C_{1-3}$alkyl or alkoxy groups, hydroxy or halogen or ($C_{1-5}$alkoxy)carbonylmethyl; and

$R^3$, $R^4$, $R^5$, $R^6$, $R^7$ and $R^8$ is each hydrogen, or

one of $R^3$, $R^4$, $R^5$, $R^6$, $R^7$ and $R^8$ is an alkyl, alkoxy, alkylthio, alkoxycarbonyl, hydroxyalkyl, alkanoyl, alkanoyloxy, alkanoylamino, carboxyalkyl or aminoalkyl group containing up to 4 carbon atoms, or a ($C_{1-2}$alkoxy)carbonyl($C_{1-2}$alkyl), mono- or di-($C_{1-2}$alkyl)amino, cyano, nitro, hydroxyl, carboxyl, amino,

mono- or di-($C_{1-2}$alkyl)amino($C_{1-2}$alkyl) or azido group or a halogen atom and the remaining five of $R^3$, $R^4$, $R^5$, $R^6$, $R^7$ and $R^8$ are each hydrogen, or

$R^3$, $R^4$ and $R^5$, are each independently hydrogen or $C_{1-3}$alkyl with the proviso that at least one is hydrogen, or one of $R^3$, $R^4$ and $R^5$ is butyl with the remaining two being hydrogen, and

$R^6$, $R^7$ and $R^8$ are each independently hydrogen or $C_{1-3}$alkyl with the proviso that at least one is hydrogen, or one of $R^6$, $R^7$ and $R^8$ is butyl with the remaining two being hydrogen;

with the proviso that when $R^1$ and $R^2$ are each independently hydrogen or straight-chained or branched $C_{1-5}$alkyl and $R^3$, $R^4$, $R^5$, $R^6$, $R^7$ and $R^8$ are all hydrogen then Z is sulphur)

and acid addition salts thereof.

Preferred compounds according to the invention include those of Formula I

wherein,

Z is oxygen or sulphur;

$R^1$ is hydrogen, $C_{1-5}$alkyl optionally substituted by fluorine, trihalomethyl, $C_{2-4}$alkenyl or alkynyl, 2-halopropen-1-yl, or $C_{2-3}$alkoxyalkyl or alkylthioalkyl;

$R^2$ is $C_{1-4}$alkyl optionaly substituted by fluorine, $C_{2-4}$ alkenyl or alkynyl, $C_{2-4}$alkoxyalkyl or alkylthioalkyl, $C_{2-3}$alkanoyl, $C_{2-4}$hydroxyalkyl, arylmethyl (wherein the aryl moiety is phenyl or thienyl and is optionally substituted by methyl, methoxy, hydroxyl or halogen), phenyl (optionally substituted by methyl, methoxy, hydroxyl or halogen) or ($C_{1-5}$alkoxy)carbonylmethyl;

$R^3$, $R^4$ and $R^5$ are each independently hydrogen or methyl, with the proviso that at least one is hydrogen, or $R^5$ is ethyl, propyl or butyl and $R^3$ and $R^4$ are hydrogen, and

$R^6$, $R^7$, and $R^8$ are each independently hydrogen or methyl, with the proviso that at least one is hydrogen, or $R^6$ is ethyl propyl or butyl and $R^7$ and $R^8$ are hydrogen,

and the salts thereof.

Particularly preferred compounds according to the invention include those of Formula I

wherein,

Z is oxygen or sulphur;

$R^1$ is hydrogen, $C_{1-4}$alkyl optionally substituted by fluorine or allyl;

$R^2$ is $C_{1-4}$alkyl optionally substituted by fluorine, allyl or benzyl; and

$R^3$, $R^4$, $R^5$, $R^6$, $R^7$ and $R^8$ are each hydrogen,

and the salts thereof.

The compounds according to the invention can be prepared by known methods or modifications thereof.

Thus viewed from a further aspect the invention provides a process for preparing a compound of Formula I or salt thereof, said process comprising at least one of the following steps:

(A) (for preparing compounds of Formula I wherein $R^2$ is other than hydrogen) cyclizing a carboxylic acid amide of general Formula II

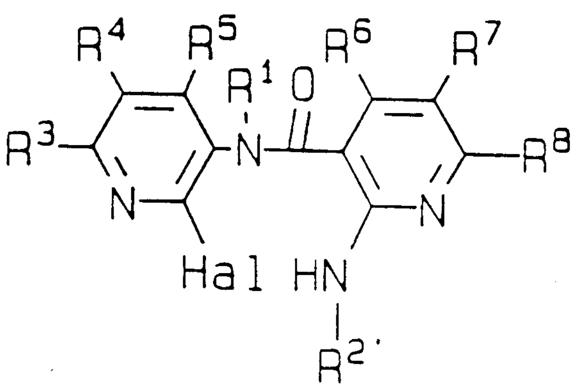

(II)

(wherein $R^1$, $R^3$, $R^4$, $R^5$, $R^6$, $R^7$ and $R^8$ are as hereinbefore defined, $R^{2'}$ is as defined for $R^2$ with the exception of hydrogen, and Hal represents a fluorine, chlorine, bromine or iodine atom);

(B) (for producing compounds of Formula I wherein $R^2$ is hydrogen) hydrolytically cleaving an arylmethyl group from a compound of Formula III

(III)

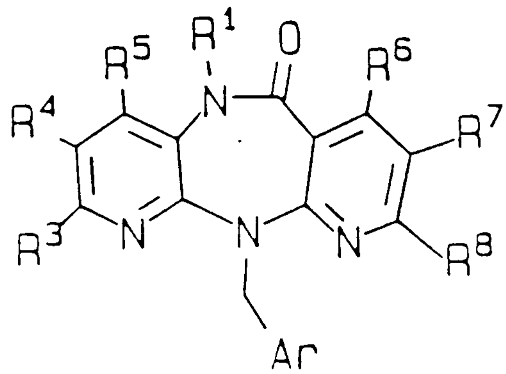

(wherein $R^1$, $R^3$, $R^4$, $R^5$, $R^6$, $R^7$ and $R^8$ are as hereinbefore defined and Ar represents an aryl group, for example, a phenyl or 4-methoxyphenyl group);

(C) (for preparing compounds of Formula I wherein $R^1$ is other than hydrogen)

converting a compound of Formula I wherein $R^1$ is hydrogen into a corresponding 5-alkali or alkaline earth metal compound and subsequently reacting said alkali or alkaline earth metal compound with a compound of Formula IV

$R^{1'}X$ (IV)

(wherein $R^{1'}$ is as hereinbefore defined for $R^1$ with the exception of hydrogen and X is the radical of a reactive ester, a halogen atom, a group $OSO_2OR^{1'}$, a methanesulphonyloxy or ethanesulphonyloxy group or an aromatic sulphonyloxy group);

(D) (for preparing compounds of Formula I wherein $R^1$ is other than hydrogen)

by reacting a compound of Formula I wherein $R^1$ is hydrogen with a compound of Formula IV (as defined above) in the presence of an amine or an alkali metal carbonate or bicarbonate;

(E) (for preparing a compound of Formula I wherein $R^2$ is other than an alkanoyl, hydroxyalkyl or alkoxycarbonylmethyl group)

converting a compound of Formula I wherein $R^2$ is hydrogen into a corresponding metal salt of Formula Va or, where $R^1$ represents hydrogen, Vb

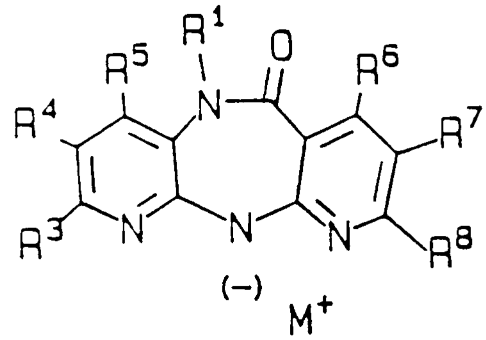

(Va)

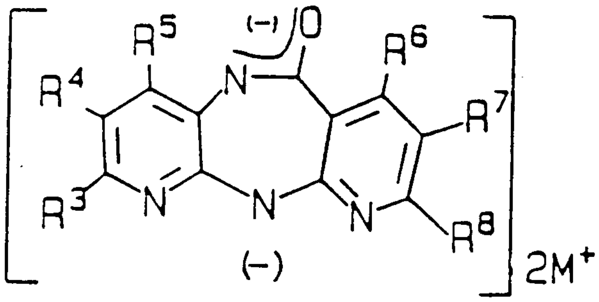

(Vb)

(wherein M represents an alkali metal, such as lithium, sodium, potassium, rubidium or cesium, or M represents the group MgHal+, wherein Hal is chlorine, bromine or iodine) and subsequently alkylating with a compound of Formula VI

$R^{2''}X$ (VI)

(wherein X is as hereinbefore defined) and $R^{2''}$ is as defined for $R^2$ with the exception of alkanoyl, hydroxyalkyl and alkoxycarbonylmethyl);

(F) (to prepare a compound of Formula I, wherein Z is sulphur)

reacting a compound of Formula I, wherein Z is oxygen, with a sulphurating agent;

(G) subsequently if desired reacting a compound so obtained to

(i) hydrolyse a nitro group to an amino group,

(ii) acylate an amino group to an alkanoylamino group,

(iii) alkylate an amino or aminoalkyl group to a mono- or di-alkylaminoalkyl group,

(iv) acylate the 11-position nitrogen where $R^1$ is hydrogen, preferably subsequent to step (E); and

(H) converting a compound of Formula I into an acid addition salt thereof or a salt of a compound of Formula I into the free base, where in any of the foregoing steps reactive groups may if desired be protected by protecting groups which are subsequently removed.

In step A, cyclisation is preferably carried out by converting the compounds of Formula II into their alkaline metal salts and subsequent condensation at temperatures between O°C and the boiling point of the reaction mixture.

If, in the starting compounds of Formula II, $R^1$ does not represent hydrogen, metallation requires at least 1 mole of the metallating agent. If $R^1$ represents hydrogen, at least 2 moles of this agent must be used. For metallation, lithium, sodium and potassium hydrides, lithium alkyls, such as n-butyl lithium, are preferably used.

The reaction is usually carried out in inert solvents, e.g. in tetrahydrofuran, 1,4-dioxane, glycoldimethyl ether, diethyleneglycoldimethyl ether, triethyleneglycoldimethyl ether, dimethylformamide, benzene or anisole. Cyclisation may also be effected by heating carboxylic acid amides of Formula II in dipolar aprotic solvents, preferably in sulfolane or dimethylsulphone. The use of catalytic quantities of strong acids, e.g. sulphuric acid, hydrochloric acid, hydrobromic acid, phosphoric acid, polyphosphoric acid, methanesulphonic acid or p-toluenesulphonic acid, has proved to be advantageous. The reaction temperature is

generally 110 to 220 °C, the preferred range of temperature being 130 to 170 °C.

In step B, hydrolysis may conveniently be effected by moderate to strong acids or Lewis-acids at temperatures of -20 to +150 °C. Such acids can be, for example, sulphuric acid, methanesulphonic acid, trifluoroacetic acid, trifluoromethanesulphonic acid, phosphoric or polyphosphoric acid. When using phosphoric or polyphosphoric acid, the addition of solvents such as benzene, toluene, phenol, anisole or veratrole has proved to be of advantage.

If Lewis acids, such as aluminium chloride or bromide are used to eliminate the arylmethyl group, solvents such as aromatic hydrocarbons, e.g. benzene, toluene, anisole, or mixtures thereof with dichloromethane are suitable.

It will be obvious to those skilled in the art that Method B is not preferred in those cases wherein any of $R^1$ and $R^3$ to $R^8$ are readily hydrolyzable substituents, for example, wherein $R^1$ is alkanoyl or any of $R^3$ to $R^8$ are alkanoylamino or alkoxycarbonyl. In cases wherein $R^1$ is alkanoyl or any of $R^3$ to $R^8$ are alkoxycarbonyl, for example, it is preferable to utilize Method A described above; when $R^1$ is hydrogen two equivalents of base must be used. In cases wherein any of $R^3$ to $R^8$ are alkanoylamino, for example, it is preferable to carry out the hydrolysis (and subsequent acylation) on the corresponding nitro derivative, and then reduce the nitro moiety to the amine, followed by acylation to yield the desired product.

In step C, the conversion of a compound of Formula I in which $R^1$ is hydrogen into the corresponding alkali metal or alkaline earth metal compound may be effected by reacting the compound of Formula I with an alkali metal or alkaline earth metal hydroxide, such as lithium hydroxide, barium hydroxide, sodium hydroxide or potassium hydroxide, with an alkali metal alcoholate, such as sodium methanolate or potassium tert-butoxide, with an alkali metal amide, such as sodium amide or potassium amide, or with an alkali metal hydride such as sodium hydride or potassium hydride. The reaction is preferably carried out at elevated temperatures and in the presence of an organic solvent. Inert organic solvents, such as tetrahydrofuran or glycoldimethyl ether are preferred if alkali metal hydrides are used as the metallating agents, whereas, if an alkali or alkaline earth metal hydroxide is used, an aqueous mixture with an organic solvent, such as methanol or tetrahydrofuran, may also be employed. For conversion of the alkali or alkaline earth metal-substituted compound thus obtained into a compound of Formula I in which $R^1$ is other than hydrogen, the solution or suspension of the alkali or alkaline earth metal compound may be reacted directly, i.e. without isolation, with a compound of Formula IV at -20 °C or at higher temperatures, up to the boiling point of the solvent or reaction medium, whichever is lower. The substitution takes place almost exclusively at the nitrogen atom in the 5-position of the compound even if $R^2$ in the starting material of Formula I is a hydrogen atom, provided that one equivalent of base and one equivalent of a compound of Formula IV are used.

It will be obvious to those skilled in the art that the presence of nucleophilic substituents in the end product may require the use of an intermediate of Formula I where $R^1$ is other than hydrogen having substituents which are, other than the 11-position nitrogen, not nucleophilic but which can be derivatized to yield the required group. For example, amino or monoalkylamino substituents at any of $R^3$ to $R^8$ are preferably obtained by alkylating or acylating such an intermediate having a nitro group at any of $R^3$ to $R^8$, and subsequently reducing the nitro group, and alkylating, if appropriate, to yield the final product.

In step D examples of amines that may be used include triethylamine, diazabicycloundecene or 4-(dimethylamino)pyridine, and examples of alkali carbonates or bicarbonates that may be used include those such as sodium and potassium carbonate or sodium bicarbonate.

In step E, the conversion of a compound of Formula I into the corresponding alkali metal compound of Formulae Va or Vb may conveniently be effected by reacting with a lithium alkyl (e.g. n-butyl lithium, or t-butyl lithium) optionally in the presence of tetramethylethylenediamine, a lithium dialkylamide, (e.g. lithium diisopropylamide, lithium dicyclohexylamide and lithium isopropylcyclohexylamide), a lithium aryl (e.g. phenyl lithium) an alkali metal hydroxide (e.g. lithium, sodium or potassium hydroxide), an alkali metal hydride (e.g. sodium or potassium hydride), an alkali metal amide (e.g. sodium or potassium amides) or a Grignard reagent (e.g. methyl magnesium iodide, ethyl magnesium bromide or phenyl magnesium bromide). One equivalent or base is required for the formation of compounds of Formula Va, whereas two equivalents of base are required for the formation of compounds of Formula Vb. The metallation is conveniently carried out in an inert organic solvent at temperatures of -78 °C and the boiling point of the reaction mixture in question. If a lithium alkyl, lithium aryl, lithium dialkylamide or Grignard reagent is used for the metallation, the preferred solvents are ethers such as tetrahydrofuran, diethyl ether or dioxane, optionally in a mixture with aliphatic or aromatic hydrocarbons, such as hexane or benzene, and the reaction may be carried out at temperatures of -20 to +80 °C. When metallation is effected with an alkali metal hydride or alkali metal amide, it is possible to use xylene, toluene, acetonitrile, dimethylformamide and dimethylsulphoxide, in addition to the solvents mentioned hereinbefore, while if an alkali metal

hydroxide is used it is also possible to use alcohols such as ethanol, methanol and aliphatic ketones such as acetone, as well as mixtures of these solvents with water.

For conversion of the alkali metal salt thus obtained into a compound of Formula I in which $R^2$ is other than an alkanoyl, hydroxyalkyl or alkoxycarbonyl methyl group, the solution or suspension of the alkali metal compound may be reacted directly, i.e. without isolation of the reaction product, with a compound of Formula VI at -20 ° C or at higher temperatures, preferably at the boiling point of the solvent or suspension medium or at the boiling point of the compound of Formula VI, whichever is lower.

It will be obvious to those skilled in the art that the presence of nucleophilic substituents in the end product may require the use of an intermediate of Formula I where $R^2$ is other than alkanoyl, hydroxyalkyl or alkoxycarbonylmethyl which has substituents other than the 11-position nitrogen, which are not nucleophilic but which can be deprotected to yield the required group. For example, amino or monoalkylamino substituents of $R^3$ to $R^8$ are preferably obtained by alkylating or acylating such an intermediate having a nitro group at any of $R^3$ to $R^8$ and subsequently reducing the nitro group, and alkylating, if appropriate, to yield the final product.

Methods (A) to (E) are particularly suited to the production of compounds of Formula I in which Z is oxygen, i.e. the diazepinone compounds.

The carboxylic acid amides of Formula II used as starting materials may be obtained, for example, by amination of 2-chloro-nicotinic amides of Formula VII

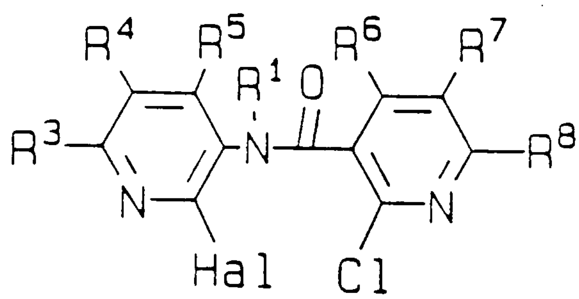

(VII)

(wherein $R^1$ to $R^8$ and Hal are as hereinbefore defined) with primary amines of Formula VIII

$H_2N\text{-}R^{2'}$ (VIII)

(wherein $R^{2'}$ is as hereinbefore defined).

The reaction can also be carried out in the presence of inorganic or organic auxiliary bases, such as triethylamine, N,N-dimethylaniline, or sodium or potassium carbonate. The reaction may be carried out without using a solvent; it is preferable, however, to use inert organic solvents at temperatures of 0 ° C to 150 ° C, preferably at reflux temprature. Suitable inert solvents include an excess of the primary amine of Formula VIII, open chain or cyclic ethers, such as tetrahydrofuran, 1,4-dioxane, glycoldimethyl ether, diethyleneglycoldimethyl ether; aromatic hydrocarbons, such as benzene, toluene, xylene, chlorobenzene or pyridine; alcohols such as methanol, ethanol, isopropanol; dipolar aprotic solvents such as dimethylformamide; 1,3-dimethyl-2-imidazolidinone, 1,3-dimethyl-tetrahydro-2(1H)-pyrimidinone and sulfolane. Starting materials of general Formula VII (wherein $R^1$ is other than hydrogen) can be prepared from 2-chloronicotinic acid amides of Formula IX

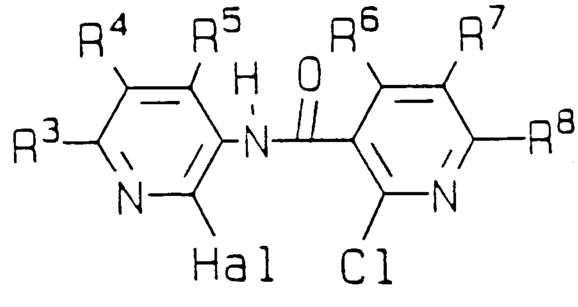

(IX)

by reaction with alklylating agents of Formula IV in the presence of proton acceptors, for example amines, such as triethylamine, diazabicycloundecene, 4-(dimethylamino)pyridine, or alkali or alkaline earth metal hydroxides, such as sodium hydroxide, potassium hydroxide, calcium hydroxide, of alkali carbonates, or alkaline earth metal carbonates or hydrogencarbonates, such as sodium carbonate or potassium carbonate, or potassium hydrogen carbonate.

2-Chloronicotinic acid amides of Formula IX can be obtained by condensation of 2-chloronicotinic acid chloride with 3-amino-2-halopyridines, under well known reaction conditions.

All the other starting materials are known from the literature or may be purchased or may be obtained by procedures known from the literature.

In step F a sulphurating agent such as 2,4-bis(4-methoxyphenyl)-1,3-dithia-2,4-diphosphetane-2,4-disulphide; bis(tricyclohexyltin)sulphide; bis(tri-n-butyltin)sulphide; bis(triphenyltin)sulphide; bis-(trimethylsilyl)sulphide or phosphorous pentasulphide may be used. The reaction is conveniently carried out in an inert organic solvent such as carbon disulphide, benzene or toluene, at ambient temperature or at an elevated temperature, preferably up to the boiling point of the reaction mixture, and preferably under anhydrous conditions. When using the above mentioned tin or silyl sulphides, it is preferable to carry out the sulphurization reaction in the presence of a Lewis acid such as boron trichloride.

It will be obvious to those skilled in the art that the presence of another carbonyl moiety in a compound of Formula I, for example, a compound wherein Z is oxygen and any of $R^3$ to $R^8$ is alkanoyl, will require that the ketone carbonyl be protected via known methods by a suitable protecting group prior to the sulphurization reaction; deprotection subsequent to the sulphurization reaction provides the desired compound. Similarly, in in cases wherein $R^2$ is, for example, alkanoyl, it will be obvious that the sulphurization reaction should be performed prior to the acylation of the 11-position nitrogen. In those cases wherein the substituents at any of $R^3$ to $R^8$ can be derived from nitro, for example, alkanoylamino, the sulphurization reaction can be performed on the corresponding nitro derivative, followed by an appropriate (known) reduction and finally acylation to yield the desired product.

Compounds of Formula I may, if desired, be converted into their acid addition salts, preferably the non-toxic, pharmaceutically acceptable acid addition salts, by conventional methods; for example, by dissolving a compound of Formula I in a suitable solvent and acidifying the solution with one or more molar equivalents of the desired acid. The invention also comprises such salts.

Examples of inorganic and organic acids which may form non-toxic, pharmaceutically acceptable acid addition salts with a compound of Formula I include the following: hydrochloric acid, hydrobromic acid, sulphuric acid, phosphoric acid, nitric acid, tartaric acid, citric acid, methanesulphonic acid, and the like. Compounds of Formula I usually. form acid addition salts with one molar equivalent of the acid.

Viewed from a further aspect the invention provides a pharmaceutical composition comprising a compound of Formula I or a physiologically acceptable acid addition thereof together at least one pharmaceutically acceptable carrier or excipient.

The compounds of Formula I or their salts may be administered in single or divided doses by oral, parenteral or topical routes. A suitable oral dosage for a compound of Formula I or a salt thereof would be in the range of about 10 to 500 mg per day. In parenteral formulations, a suitable dosage unit may contain from 1 to 50 mg of said compounds, whereas for topical administration, formulations containing 0.01 to 1% active ingredient are preferred. It should be understood, however, that the dosage administration from patient to patient will vary and the dosage for any particular patient will depend upon the clinician's judgement, who will use the size and condition of the patient as criteria for fixing a proper dosage as well as the patient's response to the drug.

When the compounds of the present invention are to be administered by the oral route, they may be administered as medicaments in the form of pharmaceutical preparations which contain them in association with a compatible pharmaceutical carrier material. Such carrier material can be an inert organic or inorganic carrier material suitable for oral administration. Examples of such carrier materials include water, gelatin, talc, starch, magnesium stearate, gum arabic, vegetable oils, polyalkylene-glycols, petroleum jelly and the like.

The pharmaceutical preparations can be prepared in a conventional manner and finished dosage forms can be solid dosage forms, for example, tablets, dragees, capsules, and the like, or liquid dosage forms, for example solutions, suspensions, emulsions and the like. The pharmaceutical preparations may be subjected to conventional pharmaceutical operations such as sterilization. Further, the pharmaceutical preparations may contain conventional adjuvants such as preservatives, stabilizers, emulsifiers, flavour-improvers, wetting agents, buffers, salts for altering the osmotic pressure and the like. Solid carrier material which can be used include, for example, starch, lactose, mannitol, methyl cellulose, microcrystalline cellulose, talc, silica, dibasic calcium phosphate, and high molecular weight polymers (such as polyethylene glycol).

For parenteral use, a compound of Formula I can be administered in an aqueous or non-aqueous solution, suspension or emulsion in a pharmaceutically acceptable oil or a mixture of liquids, which may contain bacteriostatic agents, antioxidants, preservatives, buffers or other solutes to render the solution isotonic with the blood, thickening agents, suspending agents or other pharmaceutically acceptable additives. Additives of this type include, for example, tartrate, citrate and acetate buffers, ethanol, propylene glycol, polyethylene glycol, complex formers (such as EDTA), antioxidants (such as sodium bisulphite, sodium metabisulphite and ascorbic acid), high molecular weight polymers (such as liquid polyethylene oxides) for viscosity regulation and polyethylene derivatives of sorbitol anhydrides. Preservatives may also be added if necessary, such as benzoic acid, methyl or propyl paraben, benzalkonium chloride and other quaternary ammonium compounds.

The compounds of this invention may also be administered as solutions for nasal application and may contain in addition to the compounds of this invention suitable buffers, tonicity adjusters, microbial preservatives, antioxidants and viscosity-increasing agents in an aqueous vehicle. Examples of agents used to increase viscosity are polyvinyl alcohol,cellulose derivatives, polyvinylpyrrolidone, polysorbates or glycerin. Microbial preservatives added may include benzalkonium chloride, thimerosal, chlorobutanol or phenylethyl alcohol.

Additionally, the compounds according to the invention can be administered by suppository.

The above described compounds of Formula I possess inhibitory activity against HIV reverse transcriptase. When administered in suitable dosage forms, they are useful in the prevention or treatment of AIDS, ARC and related disorders associated with HIV infection.

Viewed from a yet further aspect, the present invention provides the use of a compound of Formula I or a physiologically acceptable acid addition salt thereof for the manufacture of a therapeutic agent for combatting HIV infection.

Viewed from a still further aspect, the present invention provides a method of treatment of the human body, to combat HIV infection, said method comprising administering to said body a compound of Formula I or a physiologically acceptable acid addition salt thereof.

As stated before, the compounds provided by the invention inhibit the enzymatic activity of HIV RT. Based upon testing of these compounds, as described below, it is known that they inhibit the RNA-dependent DNA polymerase activity of HIV RT. Based upon other testing, not described herein, it is believed that they also inhibit the DNA-dependent DNA polymerase activity of HIV RT.

Utilizing the Reverse Transcriptase (RT) Assay described below, compounds can be tested for their ability to inhibit the RNA-dependent DNA polymerase activity of HIV RT. Certain specific compounds described in the Examples which appear below, were so tested. The results of this testing appears in Table I, below.

## REVERSE TRANSCRIPTASE (RT) ASSAY

Assay theory:

Among the enzymes for which Human Immunodeficiency Virus (HIV-1) encodes is a reverse transcriptase (see Benn et al. Science 230:949 (1985)), so-named because it transcribes a single-stranded DNA copy from an RNA template. This activity can be quantitatively measured in a cell-free enzyme assay which has been previously described (see Farmerie et al. Science 236:305 (1987)), and is based upon the observation that reverse transcriptase is able to use a synthetic template [poly r(C) primed with oligo d(G)] to transcribe a radio-labelled, acid-precipitable DNA strand utilizing $^3$H-dGTP as a substrate.

Materials:

a) Preparation of the enzyme Reverse transcriptase enzyme from the LAV strain of Human Immunodeficiency Virus (HIV-1) (see Benn et al. supra) is isolated from the bacterial strain JM109 (See Yanisch-Perron et al. Gene 33: 103 (1985)) expressing the DNA clone pBRTprtl+ (see Farmerie et al. supra) which is under the control of the lac promotor in the expression vector pIBI2I (International Biotechnologies Inc., New Haven, Connecticut 06535, USA). An overnight culture grown in 2XYT medium (37°C, 225 rpm) (see Maniatis et al. eds "Molecular Cloning: A laboratory manual" Cold Spring Harbor Laboratory 1982) supplemented with 100 µm/ml ampicillin for positive selection is inoculated at a 1:40 dilution into M9 medium supplemented with 10µg/ml thiamine, 0.5% casamino acids and 50 µg/ml ampicillin (see Maniatis et al. supra). The culture is incubated (37°C, 225 rpm) until it reaches an $OD^{540}$ of 0.3-0.4. At that time the repressor inhibitor IPTG (isopropyl b-D-thiogalactopyranoside) is added to 0.5mM and incubated for 2

additional hours. Bacteria are pelletted, resuspended in a 50mM Tris, 0.6mM EDTA, 0.375M NaCl buffer and digested by the addition of lysozyme (1mg/ml) for 30 minutes on ice. The cells are lysed by the addition to 0.2% NP-40 and brought to 1M NaCl.

After removal of the insoluble debris by centrifugation, the protein is precipitated by the addition of 3 volumes of saturated aqueous ammonium sulphate. The enzyme is pelleted, resuspended in RT buffer (50mM Tris pH 7.5, 1mM EDTA, 5mM DTT, 0.1M NaCl and 50% glycerol), and stored at -70 °C for further use.

b) Composition of 2X concentrated stock reaction mixture

| Stock Reagent | 2X Mix Concentration |
|---|---|
| 1M Tris pH 7.4 | 100mM |
| 1M Dithiothreitol | 40mM |
| 1M NaCl | 120mM |
| 1% Nonidet P-40 | 0.1% |
| 1M $MgCl_2$ | 4mM |
| [poly r(C)/oligo d(G)](5:1) | 2µg/ml |
| $^3$H-dGTP (81µM) | 0.6µM |

Assay Procedure:

The 2X concentrated stock reaction mixture is aliquoted and stored at -20 °C. The mixture is stable and thawed for use in each assay. This enzyme assay has been adapted to a 96 well microtiter plate system, and has been previously described (see Spira et al. J. Clinical Microbiology 25:97 (1987)). Tris buffer (50 mM, pH 7.4), vehicle (solvent diluted to match the compound dilution), or compounds in vehicle are dispensed into 96-well microtiter plates (10µl/well; 3 wells/compound). The HIV RT enzyme is thawed, diluted in 50mM Tris pH 7.4 so that 15µl of diluted enzyme contains 0.001 Unit (one Unit is that amount of enzyme needed to transform 1 micromole of substrate per minute at 25 °C), and 15µl are dispensed per well. 20µl of 0.12-0.5M EDTA are added to the first three wells of the microtiter plate. EDTA chelates the $Mg^{2+}$ present and prevents reverse transcription. This group serves as a control for the level of background polymerization which is subtracted from all other results. 25µl of the 2X reaction mixture are added to all wells and the assay is allowed to incubate at room temperature for 60 minutes. The assay is terminated by precipitating the DNA in each well with 50µl of 10% trichloroacetic acid (TCA) in 1% sodium pyrophosphate. The microtiter plate is incubated for 15 minutes at 4 °C and the precipitate is fixed onto No. 30 glass fiber paper (Schleicher & Schuell) using a Skatron semi-automatic harvester. The filters are then washed with additional 5% TCA containing 1% sodium pyrophosphate, rinsed with 70% aqueous ethanol, dried,and transferred to scintillation vials (see Spira et al. supra). Each vial receives 2 mls of scintillation cocktail and is counted in a Beckman beta counter.

Calculations for percent inhibition are as follows:

$$\%\ \text{inhibition} = \frac{\text{CPM Mean Test Value - CPM Mean Control Value X100}}{\text{CPM Mean Control Value}}$$

In order to confirm that compounds which are active in the RT Assay also have the ability to inhibit HIV replication in a living system, compounds according to the invention were also tested in the human T-cell Culture Assay described below.

HUMAN T CELL CULTURE ASSAYAssay Theory: Formation of syncytia is a feature of *in vitro* cultures of CD4+ T-cells infected with HIV-1. In this assay, T-cells are treated with a putative replication inhibiting compound and then infected with HIV-1. After incubation the culture is checked for the formation of syncytia. The absence or reduction of the number of syncytia is used as a measure of the test compound's

ability to inhibit HIV replication.

Assay Method: The target cells, designated C8166, are a subclone of human lymphoma cells of T-cell origin and are established at an initial density of $5x10^4$ per 100µl in RPMI 1640 (+ 10% fetal bovine serum) culture medium in 96 well flat bottom plates. A selected amount of test compound, dissolved in DMSO is included. After 24 hours, an excess of virus comprising 50-100 $TCID_{50}$'s ($TCID_{50}$ is the dose that results in infection in 50% of test cultures) of the HTLV-IIIB strain of HIV-1 (see Shaw et al. Science 226:1165 (1984)) are innoculated into each culture. Control cultures receive the test compound of the virus only. Four days after the viral challenge, the cultures are visually examined for the frequency and distribution of virus-induced giant cell syncytia. The percent inhibition by the test compound is determined by comparison with control values. Confirmation of the presence or absence of virus replication is accomplished by harvesting the cell free culture fluids from all experimental groups to determine the presence or absence of infectious progeny through the induction of syncytia formation in secondary human T-cell cultures after 3 days.

Regarding this assay, reference may also be had to Somasundaran et al. Science 242: 1554 (1998).

In order to assess the specificity of the enzyme inhibitory activity of the compounds provided by the invention, a few were tested, using known per se assay methods, for their ability to inhibit Feline Leukemia Virus-derived reverse transcriptase and Calf Thymus-derived DNA alpha-polymerase. None of the compounds so tested was observed to possess any inhibitory activity against these enzymes. These results indicate that the enzyme inhibitory activity of the compounds provided by the invention is directed rather specifically against HIV RT.

In order to assess approximately the cytotoxicity of the compounds provided by the invention, several such compounds were tested in the MTT Cellular Cytotoxicity Assay described below. Compounds having a relatively high $EC_{50}$ are preferred.

## MTT ASSAY FOR CELLULAR CYTOTOXICITY

Assay Theory: The MTT [3-(4,5-dimethylthiazol-2-yl)-2,5 diphenyl tetrazolium bromide] assay is based on cleavage of tetrazolium bromide by metabolically active cells, resulting in a highly quantitative blue colour. This assay has been previously described (see Mosmann, J. Immunol. Methods, 65: 55 (1983)) but has been optimized for the purposes of the testing reported herein.

Assay Method: The H9 cell line (see Jacobs, J. Natl. Cancer Inst. 34:231 (1965)), an established human lymphoma suspension cell line grown in RPMI 1640 supplemented with 10% fetal bovine serum is used as the target cell line in the assay. Cells (100µl) are plated in microtest plate wells at a concentration of $10^5$ cells per ml in the presence of varying concentrations of inhibitor. The cells are incubated at 37°C in a humidified $CO_2$ incubator. Five days later, 20µl of MTT (5mg/ml in RPMI 1640, sonicated, 0.2 micrometer filtered, and stored at 4°C) is added to each well. After 4 hours additional incubation at 37°C, 60µl of Triton-X is added to each well and throughly mixed to aid the solubilization of the crystals. Absolute ethanol (5µl) is added, the wells are incubated for 30 minutes at 60°C and immediately read on the plate reader (Dynatech) at a wavelength of 570nm.

Data from this assay are used to generate a nonlinear regression analysis which yields an value for $EC_{50}$.

The following non-limiting Examples are provided to illustrate further the present invention and enable others skilled in the art to understand it more completely. Percentages, parts and ratios are by weight unless otherwise stated.

### Example 1

5-11-Dihydro-11-ethyl-5-methyl-6H-dipyrido[3.2-b:2′,3′,-e][1,4]diazepin-6-thioneA mixture of 2.66g (0.01 mol) of 5,11-dihydro-11-ethyl-5-methyl-6H-dipyrido[3,2-b:2′3′-e][1,4]diazepin-6-one and 2.10g (0.005 mol) of Lawesson's reagent (2,4-bis(4-methoxyphenyl)-1,3-dithia-2,4-diphosphetane-2,4-disulphide) in 50ml of toluene was refluxed for 2½ hours. The solvent was then removed in vacuo and water was added to the residue. The product was extracted with ethyl acetate, dried (anhydrous sodium sulphate) and concentrated in vacuo. Purification was effected on a silica gel column using methylene chloride as the first eluent, followed by ethyl acetate/hexane (1:4 by volume). Removal of the solvent in vacuo gave 2.20g (74% of theory) of 5,11-dihydro-11-ethyl-5-methyl-6H-dipyrido[3,2-b:2′,3′-e][1,4]diazepin-6-thione as a yellow powder which was recrystallized from 10% hexane/ethyl acetate to provide 1.1g of yellow needles, m.p. 157-158°C.

The resulting compound was tested using the Reverse Transcriptase Assay (at 10µg/ml) and T Cell Culture Assay (at 3 µg/ml) and demonstrated 100% inhibition in both tests.

Example 2

5,11-Dihydro-6H-dipyrido[3,2-b:2′,3′-4][1,4]diazepin-6-one

a) 2-Chloro-N-(2-chloro-3-pyridinyl)-3-pyridinecarboxamide

In a three-necked round-bottomed flask, fitted with an efficient reflux condenser, mechanical stirrer and dropping funnel, were placed 215g (1.672 mol) of 3-amino-2-chloropyridine, dissolved in a mixture of 400ml dioxane, 500ml cyclohexane and 130ml pyridine. The solution of 299.2g (1.7 mol) of freshly prepared 2-chloro-3-pyridinecarboxylic acid chloride in 200ml dioxane was added at such a rate as to keep the vigorous reaction under control. Thereafter, the reaction mixture was allowed to cool to ambient temperature and the resulting crystalline precipitate was filtered off and washed successively with cyclohexane and ether.

The dark brown product was dissolved in 5 1 of a 3% aqueous solution of sodium hydroxide. The resulting solution was treated with charcoal, suction filtered, and the filtrate was acidified by addition of 50% aqueous acetic acid. The resulting precipitate was collected by filtration and throughly washed with water. After being dried overnight in a stream of nitrogen at ambient temperature the almost colourless product had a m.p. of 156-159 °C and was sufficiently pure for further reactions. The yield was 376.0g (84% of theory).

b) N-(2-Choro-3-pyridinyl)-2-[[(4-methoxyphenyl)methyl]amino]-3-pyridinecarboxamide

13.4g (0.05 mol) of the product obtained in step a) were dissolved in 20ml of xylene, and the resulting solution was admixed with 13.8g (0.1 mol) of p-methoxybenzylamine. Thereafter, the mixture was refluxed for two hours. The reaction mixture was then evaporated in vacuo, and the residue was purified by column chromatography on silica gel (0.2-0.5 mm) using dichloromethane/ethyl acetate 10/1 (v/v) as an eluent. Colourless crystals, melting at 122-124 °C (after recrystallization from acetonitrile) were obtained. The yield was 17.2g (93% of theory).

c) 5,11-Dihydro-11-[(4-methoxyphenyl)methyl]-6H-dipyrido-[3,2-b:2′,3′-e][1,4]diazepin-6-one

16.7g (0.0453 mol) of the product obtained in step b) were dissolved in 150ml of absolute dioxane, and the resulting solution was admixed with 6.7g (0.14 mol) of a 50% dispersion of sodium hydride in mineral oil.

Thereafter, the mixture - while protected against the external atmosphere by a low flow of nitrogen - was refluxed until no starting material could be detected by TLC. The surplus of sodium hydride was decomposed by cautious addition of 10ml of a mixture of methanol and tetrahydrofuran (50/50 v/v). The reaction mixture was neutralized by addition of acetic acid and then was evaporated in vacuo. The residue was purified by column chromatography on silica gel (0.2-0.5 mm) using successively dichloromethane/ethyl acetate 10/1 (v/v) and dichloromethane/ethyl acetate 1/1 (v/v) as eluents. The crystalline product obtained by evaporation of suitable fractions was recrystallized from acetonitrile and 2-propanol. The product has a m.p. of 213-215 °C and was identified as 5,11-dihydro-11-[(4-methoxyphenyl)methyl]-6H-dipyrido[3,2-b:2′,3′-e][1,4]diazepin-6-one. The yield was 10.3 g (68% of theory). $R_F$ 0.7 (Macherey-Nagel, Polygram$^R$ SIL G/$UV_{254}$, precoated plastic sheets for TLC; dichloromethane/ethyl acetate 1/1 (v/v)).

d) 5,11-dihydro-6H-dipyrido[3,2-b:2′,3′-e][1,4]diazepin-6-one

10.0g (0.3 mol) of the product obtained in step c) were dissolved in 50ml of trifluoroacetic acid whereby the mixture became slightly warm. Thereafter, the reaction mixture was stirred at 60 °C for 1 hour. No starting material could be detected by TLC at that time. The mixture was then evaporated in vacuo. The residue thus obtained was thoroughly stirred with 0.5% aqueous ammonia and then was filtered by suction. The raw product was recrystallized from 150ml of dimethyl sulphoxide to provide colourless crystals with m.p. of > 340 °C. The yield was 4.8g (75% of theory).

The product was identified as 5,11-dihydro-6H-dipyrido[3,2-b:2′,3′-e][1,4]-diazepin-6-one.

e) Following step c) 10 g of the resulting compound can instead be reacted with trifluoroacetic acid, stirred for one hour at room temperature, the acid removed in vacuo and the residue stirred for one hour with 0.3% ammonia. The solid was filtered and dried to give 6.7 g of the 5-methyl substituted derivative.

2.0 g of a 50% dispersion of NaH in mineral oil was added to 5.75 g of the 5-methyl derivative in 100 ml dimethylformamide. After cessation of hydrogen evolution the mixture was heated to 50 °C for 30 min. and the mixture stirred overnight at room temperature. Excess sodium hydride was decomposed by the addition of ice followed by water. The product was extracted with ether, dried (anhydrous sodium sulfate)

and evaporated to give 4.5 g of 5,-11-dihydro-11-ethyl-5-methyl-6-H dipyrido[3,2-b:2′,3′-e][1,4]diazepin-6-one.
m.p. 130-132 ° C″

Examples 3 to 54

In the following Examples, compounds of Formula I were prepared analogously to Example 2 and according to Process Steps C and D (when Z represents oxygen) and analogously to Example 1 (Process Step F) (when Z represents sulphur), and were tested using the Reverse Transcription Assay described above.

Z is an oxygen atom unless the compound is specifically indicated to be a thiolactam

$R^3$, $R^4$, $R^5$, $R^6$, $R^7$ and $R^8$ all represent hydrogen except where other substituents are shown.

| Example No. | $R^1$ | $R^2$ | $R^3$ - $R^8$ Group | Substitution | Inhibit. at 10μg/ml | M.P.°C |
|---|---|---|---|---|---|---|
| 3 | H | $-CH_2Ph$-4-OMe | - | | 81% | 209-210 |
| 4 | Me | $-CH_2Ph$ | - | | 66% | - |
| 5 | Me | $-CH_2Ph$-4-OMe | - | | 45% | 120-121.5 |
| 6 | Me | $-CH_2CH_2F$ | - | | 96% | 117-118 |
| 7 | H | -Ph | - | | 71% | 220-222 |
| 8 | H | Et | $R^5$ | Et | 100% | 212-214 |
| 9 | Me | Et | $R^7$ | Cl | 94% | 105-106 |
| 10 | Me | Et | $R^5$ | Me | 98% | 157-159 |
| 11 | Me | $-COCH_3$ | - | | 73% | 138-143 |
| 12 | $-CH_2SCH_3$ | Et | - | | 93% | 118-120 |
| 13 | H | Et | $R^3$,$R^4$ | Me | 100% | 212-214 |
| 14 | H | Et | $R^8$ | Me | 86% | 244-247 |
| 15 | H | Et | $R^3$ | Me | 91% | 263-266 |
| 16 | H | Et | $R^3$,$R^5$ | Me | 100% | 210-211 |
| 17 | H | Et | $R^4$ | Me | 95% | not avail. |
| 18 | Me | Et | $R^4$ | Me | 96% | 94-96 |
| 19 | H | $-COCH_3$ | - | | 60% | >215(dec) |
| 20 | Me | Et | $R^3$,$R^8$ | Me | 98% | 100-102 |
| 21 | H | Et | $R^3$,$R^5$,$R^8$ | Me | 100% | 228-230 |
| 22 | Me | Et | $R^3$ | Me | 100% | 124-126 |
| 23 | H | Et | $R^4$,$R^5$ | Me | 100% | 265-266 |
| 24 | Me | Et | $R^4$,$R^5$ | Me | 99% | 119-120 |
| 25 | Me | Et | $R^8$ | Me | 96% | 79-93 |
| 26 | $-COCH_3$ | Et | - | | 96% | 123-124.5 |

| Example No. | $R^1$ | $R^2$ | $R^3$-$R^8$ Group | Substitution | Inhib. at 10$\mu$g/ml | M.P.°C |
|---|---|---|---|---|---|---|
| 27 | Me | $-CH_2SCH_3$ | - | | 99% | 109-110 |
| 28 | H | Et | $R^4$ | Cl | 92% | 217-218 |
| 29 | Me | Et | $R^4$ | Cl | 99% | 124-125 |
| 30 | $-CH_2Ph$ | $-COCH_3$ | - | | 37% | 169-170 |
| 31 | Me | $-CH_2CH=CH_2$ | - | | 99% | 93-95 |
| 32 | H | Et | $R^3$ | Cl | 96% | 252-254 |
| 33 | Me | Et | - | | 100% | 143-145 |
| 34 | Me | $-CH_2C\equiv CH$ | - | | 94% | 169-170 |
| 35 | Me | Et | $R^3$ | Cl | 100% | 125-126 |
| 36 | H | Et | $R^6$ | Me | 80% | 193-194 |
| 37 | H | Et | $R^7$,$R^8$ | Me | 42% | 204-206 |
| 38 | H | Et | $R^7$ | Me | 89% | 182-183 |
| 39 | H | Et | $R^5$ | Cl | 100% | 184-186 |
| 40 | H | Et | $R^5$ | OMe | 96% | 156-157 |
| 41 | H | Et | $R^5$ | Et | 100% | 218-219 |
| 42 | H | Et | $R^7$ | Br | - | 233.5-235.5 |
| 43 | Me | Et | $R^4$ | $NO_2$ | - | 154-156 |
| 44 | Me | Et | $R^4$ | $NH_2$ | - | 235-240 |
| 45 | H | i-Pr | $R^5$ | Me | - | 188-189 |
| 46 | Me | Et | $R^3$,$R^5$ | Me | - | 151-153 |
| 47 | Me | Et | $R^6$ | Me | - | 122-124 |
| 48 * | H | Et | $R^5$ | Me | - | 158-159 |
| 49 ‡ | H | Et | $R^5$ | OH | - | 295-296 |
| 50 | Me | Et | $R^3$ | OMe | - | 116-118 |

| | | | | | | |
|---|---|---|---|---|---|---|
| 51 | Me | Et | $R^3$ | OH | - | 215-218 |
| 52 * | H | Et | $R^3,R^5$ | Me | - | 199-201 |
| 53 | Me | Et | $R^3$ | $NH_2$ | - | 197-199 |
| 54 | Me | Et | $R^3$ | NHMe | - | 186-189 |

* compound is a thiolactam (Z represents sulphur)
ǂ 2HBr salt

Example A

Capsules or Tablets

Composition A1

| Ingredients | Quantity |
|---|---|
| Active Compound | 50 mg |
| Starch | 160 mg |
| Microcrystalline Cellulose | 90 mg |
| Sodium Starch Gluctate | 10 mg |
| Magnesium Stearate | 2 mg |
| Fumed colloidal silica | 1 mg |

Composition A2

| Ingredients | Quantity |
|---|---|
| Active compound | 50mg |
| Dicalcium Phosphate | 160mg |
| Microcrystalline Cellulose | 90 mg |
| Stearic acid | 5 mg |
| Sodium Starch Glycolate | 10 mg |
| Fumed colloidal silica | 1 mg |

The compound of Example 2 is blended into a powder mixture with the premixed excipient materials identified above with the exception of the stearic acid or magnesium stearate lubricant. The lubricant is then blended in and the resulting blend is compressed into tablets or filled into hard gelatin capsules.

Example B

| Parenteral Solutions | |
|---|---|
| Ingredients | Quantity |
| Active compound | 500 mg |
| Tartaric acid | 1.5 g |
| Benzyl Alcohol | 0.1% by weight |
| Water for injections | q.s. to 100 ml |

The excipient materials are mixed with the water and the active compound is then added. Mixing is continued until the solution is clear. The pH of this solution is adjusted to 3.0 and it is then filtered into the appropriate vials or ampoules and sterilized by autoclaving.

Example C

| Nasal Solutions | |
|---|---|
| Ingredients | Quantity |
| Active Compound | 100 mg |
| Citric acid | 1.92 g |
| Benzalkonium chloride | 0.025% by weight |
| EDTA | 0.1% by weight |
| Polyvinylalcohol | 10% by weight |
| Water | q.s. to 100 ml |

The excipient materials are mixed with water and the active compound is then added. Mixing is continued until the solution is clear. The pH of this solution is adjusted to 4.0 and it is then filtered into the appropriate vials or ampoules.

**Claims**

**1.** A compound of Formula I

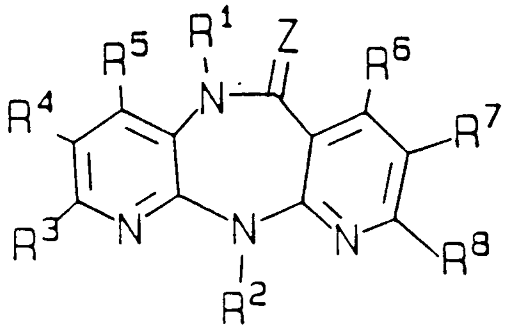

(I)

(wherein,
Z is oxygen or sulphur;
$R^1$ is hydrogen, $C_{1-5}$alkyl optionally substituted by fluorine, trihalomethyl, $C_{3-5}$alkenyl or alkynyl, 2-halopropen-1-yl, arylmethyl (wherein the aryl moiety is phenyl, thienyl or furanyl and is optionally substituted by methyl, methoxy or halogen), $C_{2-3}$ alkanoyl or $C_{2-4}$ alkoxyalkyl or alkylthioalkyl;
$R^2$ is hydrogen, $C_{1-5}$ alkyl optionally substituted by fluorine, $C_{2-5}$alkenyl or alkynyl, $C_{2-4}$alkoxyalkyl or alkylt hioalkyl, $C_{2-4}$alkanoyl, $C_{2-5}$hydroxyalkyl, arylmethyl (where in the aryl moiety is phenyl, thienyl or furanyl, and is optionally substituted by $C_{1-3}$ alkyl or alkoxy, hydroxyl or halogen), phenyl optionally substituted by $C_{1-3}$alkyl or alkoxy groups, hydroxy or halogen or ($C_{1-5}$alkoxy)carbo nylmethyl; and
$R^3$, $R^4$, $R^5$, $R^6$, $R^7$ and $R^8$ is each hydrogen, or
one of $R^3$, $R^4$, $R^5$, $R^6$, $R^7$ and $R^8$ is an alkyl, alkoxy, alkylthio, alkoxycarbonyl, hydroxyalkyl, alkanoyl, alkanoyloxy, alkanoylamino, carboxyalkyl or aminoalkyl group containing up to 4 carbon atoms, or a ($C_{1-2}$alkoxy)carbonyl($C_{1-2}$alkyl), mono- or di-($C_{1-2}$alkyl)amino, cyano, nitro, hydroxyl, carboxyl, amino, mono- or di-($C_{1-2}$alkyl)amino($C_{1-2}$alkyl) or azido gro up or a halogen atom and the remaining five of $R^3$, $R^4$, $R^5$, $R^6$, $R^7$ and $R^8$ are each hydrogen, or
$R^3$, $R^4$ and $R^5$, are each independently hydrogen or $C_{1-3}$alkyl with the proviso that at least one is hydrogen, or one of $R^3$, $R^4$ and $R^5$ is butyl with the remaining two being hydrogen, and
$R^6$, $R^7$ and $R^8$ are each independently hydrogen or $C_{1-3}$alkyl with the proviso that at least one is hydrogen, or one of $R^6$, $R^7$ and $R^8$ is butyl with the remaining two being hydrogen;

with the proviso that when $R^1$ and $R^2$ are each independently hydrogen or straight-chained or branched $C_{1-5}$alkyl and $R^3$, $R^4$, $R^5$, $R^6$, $R^7$ and $R^8$ are all hydrogen then Z is sulphur)
or an acid addition salts thereof.

**2.** A compound of Formula I, as claimed in claim 1, whe rein,
Z is oxygen or sulphur;
$R^1$ is hydrogen, $C_{1-5}$alkyl optionally substituted by fluori ne, trihalomethyl, $C_{2-4}$alkenyl or alkynyl, 2-halo-propen-1-yl,or $C_{2-3}$alkoxyalkyl or alkylthioalkyl;
$R^2$ is $C_{1-4}$alkyl optionaly substituted by fluorine, $C_{2-4}$ alk enyl or alkynyl, $C_{2-4}$alkoxyalkyl or alkylthioalkyl, $C_{2-3}$alkanoyl, $C_{2-4}$hydroxyalkyl, arylmethyl (wherein the ary l moiety is phenyl or thienyl and is optionally substituted by methyl, methoxy, hydroxyl or halogen), phenyl (optionally substituted by methyl, methoxy, hydroxyl or halogen) or ($C_{1-5}$alkoxy)carbonylmethyl;
$R^3$, $R^4$ and $R^5$ are each independently hydrogen or methyl, with the proviso that at least one is hydrogen, or $R^5$ is ethyl, propyl or butyl and $R^3$ and $R^4$ are hydrogen, and
$R^6$, $R^7$, and $R^8$ are each independently hydrogen or methyl, with the proviso that at least one is hydrogen, or $R^6$ is ethyl, propyl or butyl and $R^7$ and $R^8$ are hydrogen,
or an acid addition salt thereof.

**3.** A compound of Formula I as claimed in claim 1, wherein,
Z is oxygen or sulphur;
$R^1$ is hydrogen, $C_{1-4}$alkyl optionally substituted by fluori ne or allyl;
$R^2$ is $C_{1-4}$alkyl optionally substituted by fluorine, allyl or benzyl; and
$R^3$, $R^4$, $R^5$, $R^6$, $R^7$ and $R^8$ are each hydrogen,
or an acid addition salt thereof.

**4.** A compound as claimed in any one of claims 1 to 3 being a physiologically acceptable acid addition salt of a compound of Formula I (as defined in claim 1).

**5.** A pharmaceutical composition comprising a compound of Formula I or a physiologically acceptable acid additi on salt thereof as claimed in any of claims 1 to 4 together with at least one pharmaceutically acceptable carrier or excipient.

**6.** A process for preparing a compound as defined in any of claims 1 to 4, said process comprising at least one of the following steps:
(A) (for preparing compounds of Formula I wherein $R^2$ is other than hydrogen) cyclizing a carboxylic acid amide of general Formula II

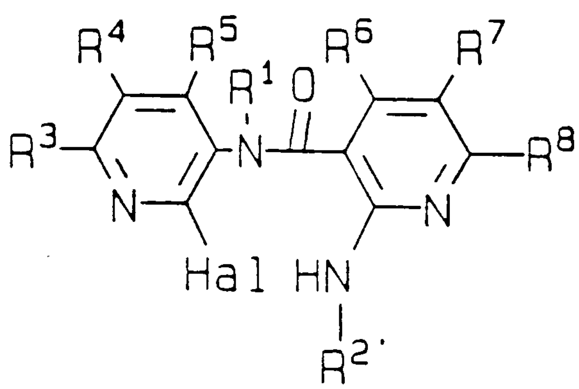

(II)

(wherein $R^1$, $R^3$, $R^4$, $R^5$, $R^6$, $R^7$ and $R^8$ are as hereinbefore defined, $R^{2'}$ is as defined for $R^2$ with the exception of hydrogen, and Hal represents a fluorine, chlorine, bromine or iodine atom);
(B) (for producing compounds of Formula I wherein $R^2$ is hydrogen) hydrolytically cleaving an arylmethyl group from a compound of Formula III

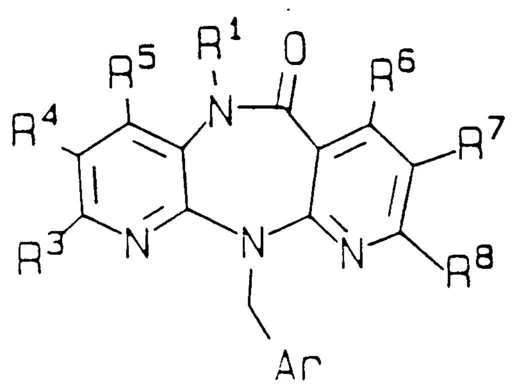

(III)

(wherein $R^1$, $R^3$, $R^4$, $R^5$, $R^6$, $R^7$ and $R^8$ are as hereinbefore defined and Ar represents an aryl group);

(C) (for preparing compounds of Formula I wherein $R^1$ is other than hydrogen)

converting a compound of Formula I wherein $R^1$ is hydrogen into a corresponding 5-alkali or alkaline earth metal compound and subsequently reacting said alkali or alkali ne earth metal compound with a compound of Formula IV

$R^{1'}X$ (IV)

(wherein $R^{1'}$ is as hereinbefore defined for $R^1$ with the exception of hydrogen and X is the radical of a reactive ester, a halogen atom, a group $OSO_2OR^{1'}$, a methanesulphonyloxy or ethanesulphonyloxy group or an aromatic sulphonyloxy group);

(D) (for preparing compounds of Formula I wherein $R^1$ is other than hydrogen)

by reacting a compound of Formula I wherein $R^1$ is hydrogen with a compound of Formula IV (as defined above) in the presence of an amine or an alkali metal carbonate or bicarbonate;

(E) (for preparing a compound of Formula I wherein $R^2$ is other than an alkanoyl, hydroxyalkyl or alkoxycarbonylmethyl group)

converting a compound of Formula I wherein $R^2$ is hydrogen into a corresponding metal salt of Formula Va or, where $R^1$ represents hydrogen, Vb

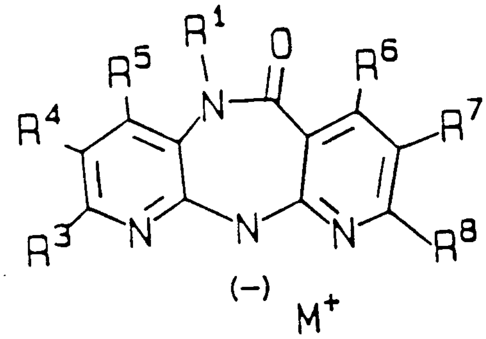

(Va)

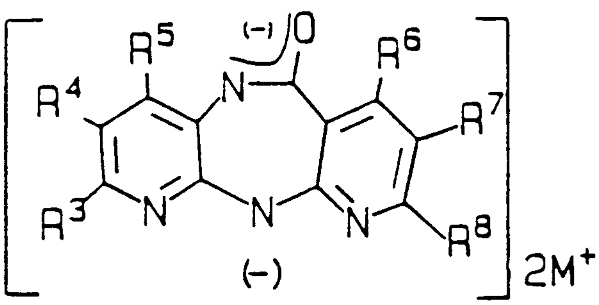

(Vb)

(wherein M represents an alkali metal, such as lithium, sodium, potassium, rubidium or cesium, or M represents the group MgHal+, wherein Hal is chlorine, bromine or iodine) and subsequently alkylating with a compound of Formula VI

$R^{2''}X$ (VI)

(wherein X is as hereinbefore defined) and $R^{2''}$ is as defined for $R^2$ with the exception of alkanoyl, hydroxyalkyl and alkoxycarbonylmethyl);
(F) (to prepare a compound of Formula I, wherein Z is sulphur)
reacting a compound of Formula I, wherein Z is oxygen, with a sulphurating agent.

7. Use of a compound of Formula I or a physiologically acceptable acid addition salt thereof as claimed in any of claims 1 to 4 for the manufacture of a therapeutic agent for combatting HIV infection.

8. A compound of Formula I as claimed in any of claims 1 to 3 or a physiologically acceptable acid addition salt thereof as an active pharmaceutical substance.

## Patentansprüche

1. Verbindung der Formel I

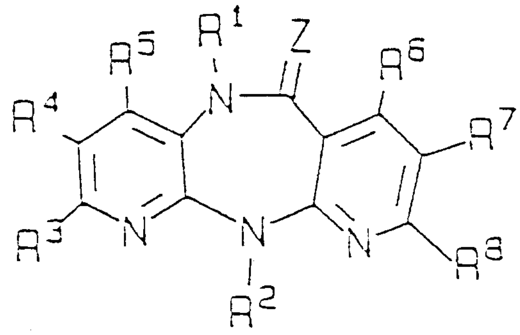

(I),

worin bedeuten:

| | |
|---|---|
| Z | Sauerstoff oder Schwefel; |
| $R^1$ | Wasserstoff, gegebenenfalls durch Fluor oder Trihalogenmethyl substituiertes $C_{1-5}$-Alkyl, $C_{3-5}$-Alkenyl oder -Alkinyl, 2-Halogenpropen-1-yl, Arylmethyl (wobei der Arylanteil eine Phenyl-, Thienyl- oder Furanylgruppe ist und gegebenenfalls durch Methyl, Methoxy oder Halogen substituiert ist), $C_{2-3}$-Alkanoyl oder $C_{2-4}$-Alkoxyalkyl oder -Alkylthioalkyl; |
| $R^2$ | Wasserstoff, gegebenfalls duch Fluor substituiertes $C_{1-5}$-Alkyl, $C_{2-5}$-Alkenyl oder -Alkinyl, $C_{2-4}$-Alkoxyalkyl oder -Thioalkyl $C_{2-4}$-Alkanoyl, $C_{2-5}$-Hydroxyalkyl, Arylmethyl (wobei der Arylanteil eine Phenyl-, Thienyl oder Furanylgruppe ist und gegebenenfalls durch $C_{1-3}$-Alkyl- oder - Alkoxygruppen, Hydroxy oder Halogen substituiert ist), gegebenenfalls durch $C_{1-3}$-Alkyl- oder -Alkoxygruppen, Hydroxy oder Halogen substituiertes Phenyl oder ($C_{1-5}$-Alkoxy)-carbonylmethyl; und |
| $R^3$, $R^4$, $R^5$, $R^6$, $R^7$ und $R^8$ | sämtlich Wasserstoff oder eines der Symbole $R^3$, $R^4$, $R^5$, $R^6$, $R^7$ und $R^8$ eine Alkyl-, Alkoxy-, Alkylthio-, Alkoxycarbonyl-, Hydroxyalkyl-, Alkanoyl-, Alkanoyloxy-, Alkanoylamino-, Carboxyalkyl oder Aminoalkylgruppe mit bis zu 4 Kohlenstoffatomen, oder eine ($C_{1-2}$-Alkoxy)-carbonyl($C_{1-2}$-Alkyl)-,Mono- oder Di($C_{1-2}$-alkyl)amino-, Cyano-, Nitro-, Hydroxy-, Carboxy-, Amino-, Mono- oder Di-($C_{1-2}$-Alkyl)amino($C_{1-2}$-Alkyl)- oder Azidogruppe oder ein Halogenatom und die verbleibenden fünf der Symbole $R^3$, $R^4$, $R^5$, $R^6$, $R^7$ und $R^8$ jeweils für Wasserstoff stehen, oder |
| $R^3$, $R^4$ und $R^5$ | unabhängig voneinander Wasserstoff oder $C_{1-3}$-Alkyl, mit der Massgabe, dass wenigstens eines der Symbole Wasserstoff bedeutet, oder eines der Symbole $R^3$, $R^4$ und $R^5$ für Butyl steht und die verbleibenden zwei Symbole Wasserstoff darstellen, und |
| $R^6$, $R^7$ und $R^8$ | unabhängig voneinander Wasserstoff oder $C_{1-3}$-Alkyl, mit der Massgabe, dass wenigstens eines der Symbole Wasserstoff bedeutet, oder eines der Symbole $R^6$, $R^7$ und $R^8$ für Butyl steht und die verbleibenden zwei Symbole Wasserstoff darstellen; und |

mit der Massgabe, dass Z Schwefel bedeutet, wenn $R^1$ und $R^2$ unabhängig voneinander Wasserstoff oder geradkettiges oder verzweigtes $C_{1-5}$-Alkyl darstellen und die Symbole $R^3$, $R^4$, $R^5$, $R^6$, $R^7$ und $R^8$ sämtlich für Wasserstoff stehen,
oder ein Säureadditionssalz dieser Verbindung.

2. Verbindung der Formel I nach Anspruch 1, worin

| | |
|---|---|
| Z | Sauerstoff oder Schwefel; |
| $R^1$ | Wasserstoff, gegebenenfalls durch Fluor oder Trihalogenmethyl substituiertes $C_{1-5}$-Alkyl, $C_{2-4}$-Alkenyl oder -Alkinyl, 2-Halogenpropen-1-yl oder $C_{2-4}$-Alkoxyal- |

kyl oder -Alkylthioalkyl;

$R^2$ Wasserstoff, gegebenfalls duch Fluor substituiertes $C_{1-4}$-Alkyl, $C_{2-4}$-Alkenyl oder -Alkinyl, $C_{2-4}$-Alkoxyalkyl oder -Alkylthioalkyl, $C_{2-3}$-Alkanoyl, $C_{2-4}$-Hydroxyalkyl, Arylmethyl (wobei der Arylanteil eine Phenyl- oder Thienylgruppe ist und gegebenenfalls durch Methyl, Methoxy, Hydroxy oder Halogen substituiert ist) oder ($C_{1-5}$-Alkoxy)carbonylmethyl;

$R^3$, $R^4$ und $R^5$ unabhängig voneinander Wasserstoff oder Methyl, mit der Massgabe, dass wenigstens eines der Symbole Wasserstoff bedeutet, oder $R^5$ für Ethyl, Propyl oder Butyl steht und $R^3$ und $R^4$ Wasserstoff darstellen, und

$R^6$, $R^7$ und $R^8$ unabhängig voneinander Wasserstoff oder Methyl, mit der Massgabe, dass wenigstens eines der Symbole Wasserstoff bedeutet, oder $R^6$ für Ethyl, Propyl oder Butyl steht und $R^7$ und $R^8$ Wasserstoff darstellen,

bedeuten, oder ein Säureadditionssalz dieser Verbindung.

**3.** Verbindung der Formel I gemäss Anspruch 1, worin

Z Sauerstoff oder Schwefel;

$R^1$ Wasserstoff, gegebenenfalls durch Fluor substituiertes $C_{1-4}$-Alkyl oder Allyl;

$R^2$ Wasserstoff, gegebenfalls duch Fluor substituiertes $C_{1-4}$-Alkyl, Allyl oder Benzyl; und

$R^3$, $R^4$, $R^5$, $R^6$, $R^7$ und $R^8$ sämtlich Wasserstoff

bedeuten oder ein Säureadditionssalz dieser Verbindung.

**4.** Verbindung nach irgendeinem der Ansprüche 1 bis 3, welche ein physiologisch annehmbares Säureadditionssalz einer Verbindung der Formel I (wie in Anspruch 1 beansprucht) ist.

**5.** Pharmazeutische Zubereitung, enthaltend eine Verbindung der Formel I oder ein physiologisch annehmbares Säureadditionssalz dieser Verbindung, wie in einem der Ansprüche 1 bis 4 beansprucht, zusammen mit mindestens einem pharmazeutisch annehmbaren Trägerstoff oder Arzneimittelträger.

**6.** Verfahren zur Herstellung einer Verbindung, wie in einem der Ansprüche 1 bis 4 definiert, wobei das Verfahren mindestens eine der nachstehend aufgeführten Verfahrensstufen umfasst:

(A) Cyclisierung eines Carbonsäureamides der allgemeinen Formel II

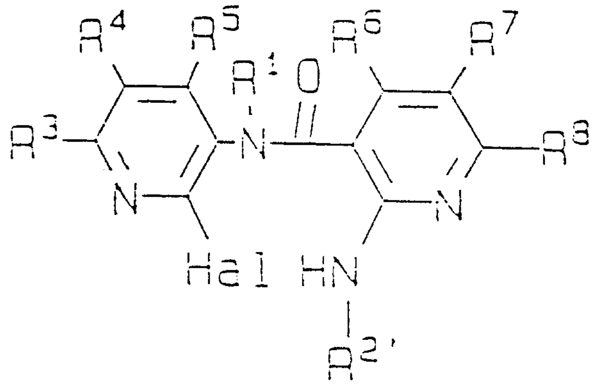

(II)

(worin $R^1$, $R^3$, $R^4$, $R^5$, $R^6$, $R^7$ und $R^8$ wie vorstehend definiert sind, $R^{2'}$ die für $R^2$ angegebenen Bedeutungen mit Ausnahme von Wasserstoff hat und Hal ein Fluor-, Chlor-, Brom oder Jodatom darstellt) zur Herstellung von Verbindungen der Formel I, worin $R^2$ eine andere Bedeutung als Wasserstoff hat;

(B) Abspaltung einer Arylmethylgruppe aus einer Verbindung der Formel III

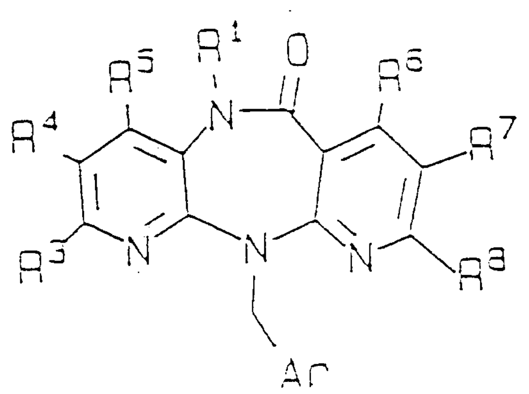

(III)

(worin $R^1$, $R^3$, $R^4$, $R^5$, $R^6$, $R^7$ und $R^8$ wie vorstehend definiert sind und Ar eine Arylgruppe darstellt) zur Herstellung von Verbindungen der Formel I, worin $R^2$ für Wasserstoff steht;

(C) Umwandlung einer Verbindung, worin $R^1$ Wasserstoff bedeutet, in eine entsprechende 5-Alkali- oder -Erdalkalimetallverbindung und nachfolgende Umsetzung dieser Alkali- oder Erdalkalimetallverbindung mit einer Verbindung der Formel IV

$R^{1'}$ (IV)

(worin $R^{1'}$ die vorstehend für $R^1$ angegebenen Bedeutungen mit Ausnahme von Wasserstoff hat und X das Radikal eines reaktiven Esters, ein Halogenatom, eine Gruppe $OSO_2OR^{1'}$, eine Methansulfonyloxy- oder Ethansulfonyloxygruppe oder eine aromatische Sulfonyloxygruppe bedeutet) zur Herstellung einer Verbindung der Formel I, worin $R^1$ eine andere Bedeutung als Wasserstoff hat;

(D) Umsetzung einer Verbindung der Formel I, worin $R^1$ Wasserstoff bedeutet, mit einer Verbindung der Formel IV, wie vorstehend definiert, in Gegenwart eines Amins oder eines Alkalimetallcarbonates oder -bicarbonates zur Herstellung einer Verbindung der Formel I, worin $R^1$ eine andere Bedeutung als Wasserstoff hat;

(E) Umwandlung einer Verbindung der Formel I, worin $R^2$ Wasserstoff bedeutet, in ein entsprechendes Metallsalz der Formel (Va) oder, sofern $R^1$ Wasserstoff bedeutet, ein entsprechendes Metallsalz der Formel (Vb)

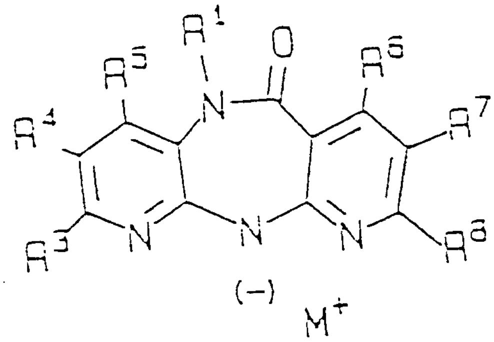

(Va)

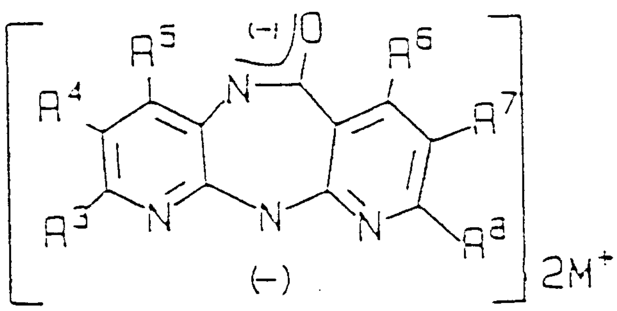

(Vb)

(worin M für ein Alkalimetall, wie Lithium, Natrium Kalium, Rubidium oder Cäsium, oder die Gruppe $MgHal^+$, worin Hal Chlor, Brom oder Jod bedeutet, steht) und nachfolgende Alkylierung mittels einer Verbindung der Formel VI

$R^{2''}X$ (VI)

(worin X wie im Vorhegehenden definiert ist und $R^{2''}$ die für $R^2$ angegebenen Bedeutungen mit Ausnahme von Alkanoyl, Hydroxyalkyl und Alkoxycarbonylmethyl hat) zur Herstellung einer Verbindung der Formel I, worin $R^2$ eine andere Bedeutung als Alkanoyl, Hydroxyalkyl oder Alkoxycarbonylmethyl hat;

(F) Umsetzung einer Verbindung der Formel I, worin Z für Sauerstoff steht, mit einem Sulfurierungsreagens zur Herstellung einer Verbindung der Formel I, worin Z Schwefel bedeutet.

**7.** Verwendung einer Verbindung der Formel I oder eines ihrer physiologisch annehmbaren Saureadditionssalze, wie in einem der Ansprüche 1 bis 4 beansprucht, zur Herstellung eines Heilmittels zur Bekämpfung von HIV-Infektionen.

**8.** Verbindung der Formel I, wie in einem der Ansprüche 1 bis 3 beansprucht, oder eines ihrer physiologisch annehmbaren Säureadditionssalze als pharmazeutischer Wirkstoff.

## Revendications

**1.** Composé de formule I

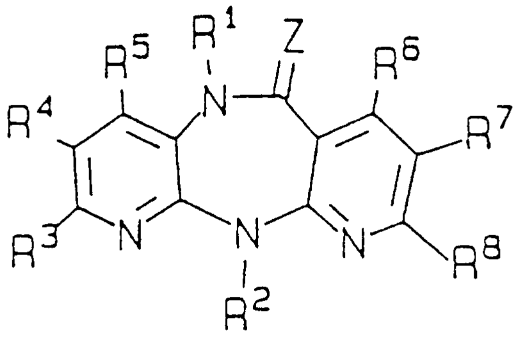

(I)

dans laquelle

Z est l'oxygène ou le soufre;

$R^1$ est l'hydrogène, un alkyle en $C_{1-5}$ éventuellement substitué par le fluor, un trihalogénométhyle, un alcényle en $C_{3-5}$ ou un alcynyle en $C_{3-5}$, un 2-halogénopropén-1-yle, un arylméthyle (dans lequel la partie aryle est un phényle, thiényle ou furanyle et est éventuellement substituée par méthyle, méthoxy ou halogène), un alcanoyle en $C_{2-3}$ ou alcoxyalkyle ou alkylthioalkyle en $C_{2-4}$;

$R^2$ est l'hydrogène, un alkyle en $C_{1-5}$ éventuellement substitué par le fluor, un alcényle en $C_{2-5}$ ou un alcynyle en $C_{2-5}$, un alcoxyalkyle en $C_{2-4}$ ou un alkylthioalkyle en $C_{2-4}$, un alcanoyle en $C_{2-4}$, un hydroxyalkyle en $C_{2-5}$, un arylméthyle (dans lequel la partie aryle est un phényle, thiényle ou furanyle, et est éventuellement substituée par alkyle ou alcoxy en $C_{1-3}$, hydroxyle ou halogène), un phényle éventuellement substitué par des groupes alkyle ou alcoxy en $C_{1-3}$, hydroxy ou halogène ou (alcoxy en $C_{1-5}$)carbonylméthyle; et

$R^3$, $R^4$, $R^5$, $R^6$, $R^7$ et $R^8$ sont chacun l'hydrogène, ou l'un des groupes $R^3$, $R^4$, $R^5$, $R^6$, $R^7$ et $R^8$ est un groupe alkyle, alcoxy, alkylthio, alcoxycarbonyle, hydroxyalkyle, alcanoyle, alcanoyloxy, alcanoylamino, carboxyalkyle ou aminoalkyle contenant jusqu'à 4 atomes de carbone, ou un groupe (alcoxy en $C_{1-2}$)carbonyl(alkyle en $C_{1-2}$), mono- ou di(alkyle en $C_{1-2}$)amino, cyano, nitro, hydroxyle, carboxyle, amino, mono- ou di-(alkyle en $C_{1-2}$)amino(alkyle en $C_{1-2}$) ou azido ou un atome d'halogène et les cinq groupes restants parmi $R^3$, $R^4$, $R^5$, $R^6$, $R^7$ et $R^8$ sont chacun l'hydrogène, ou bien

$R^3$, $R^4$ et $R^5$ sont chacun indépendamment l'hydrogène ou un alkyle en $C_{1-3}$ à condition que l'un au moins soit l'hydrogène, ou bien l'un des groupes $R^3$, $R^4$ et $R^5$ est un butyle et les deux autres sont l'hydrogène, et

$R^6$, $R^7$ et $R^8$ sont chacun indépendamment l'hydrogène ou un alkyle en $C_{1-3}$ à condition que l'un au moins soit l'hydrogène, ou bien l'un des groupes $R^6$, $R^7$ et $R^8$ est un butyle et les deux autres sont l'hydrogène;

à condition que lorsque $R^1$ et $R^2$ sont chacun indépendamment l'hydrogène ou un alkyle en $C_{1-5}$ linéaire ou ramifié et $R^3$, $R^4$, $R^5$, $R^6$, $R^7$ et $R^8$ sont tous l'hydrogène, Z soit le soufre

ou l'un de ses sels d'addition d'acide.

**2.** Composé de formule I selon la revendication 1, dans lequel

Z est l'oxygène ou le soufre;

$R^1$ est l'hydrogène, un alkyle en $C_{1-5}$ éventuellement substitué par le fluor, un trihalogénométhyle, un alcényle en $C_{3-5}$ ou un alcynyle en $C_{3-5}$, un 2-halogénopropén-1-yle ou un alcoxyalkyle ou alkylthioalkyle en $C_{2-3}$;

$R^2$ est un alkyle en $C_{1-4}$ éventuellement substitué par le fluor, un alcényle en $C_{2-4}$ ou un alcynyle en $C_{2-4}$, un alcoxyalkyle en $C_{2-4}$ ou un alkylthioalkyle en $C_{2-4}$, un alcanoyle en $C_{2-3}$, un hydroxyalkyle en $C_{2-4}$, un arylméthyle (dans lequel la partie aryle est un phényle ou un thiényle et est éventuellement substituée par un méthyle, méthoxy, hydroxyle ou un halogène), un phényle (éventuellement substitué par méthyle, méthoxy, hydroxyle ou halogène) ou un (alcoxy en $C_{1-5}$)-carbonylméthyle;

$R^3$, $R^4$ et $R^5$ sont chacun indépendamment l'hydrogène ou un méthyle, à condition que l'un au moins soit l'hydrogène, ou bien $R^5$ est un groupe éthyle, propyle ou butyle et $R^3$ et $R^4$ sont

l'hydrogène, et

$R^6$, $R^7$ et $R^8$ sont chacun indépendamment l'hydrogène ou un méthyle, à condition que l'un au moins soit l'hydrogène, ou bien $R^6$ est un éthyle, propyle ou butyle et $R^7$ et $R^8$ sont l'hydrogène,

ou l'un de ses sels d'addition d'acide.

**3.** Composé de formule I selon la revendication 1, dans lequel

Z est l'oxygène ou le soufre;

$R^1$ est l'hydrogène, un alkyle en $C_{1-4}$ éventuellement substitué par le fluor ou un allyle;

$R^2$ est alkyle en $C_{1-4}$ éventuellement substitué par le fluor, un allyle ou un benzyle; et

$R^3$, $R^4$, $R^5$, $R^6$, $R^7$ et $R^8$ sont chacun l'hydrogène,

ou l'un de ses sels d'addition d'acide.

**4.** Composé selon l'une quelconque des revendications 1 à 3 qui est un sel d'addition d'acide acceptable du point de vue physiologique d'un composé de formule I (tel que défini dans la revendication 1).

**5.** Composition pharmaceutique comprenant un composé de formule I ou l'un de ses sels d'addition d'acide acceptables du point de vue physiologique selon l'une quelconque des revendications 1 à 4 ainsi qu'au moins un véhicule ou excipient acceptable du point de vue pharmaceutique.

**6.** Procédé de préparation d'un composé selon l'une quelconque des revendications 1 à 4, ledit procédé comprenant au moins l'une des étapes suivantes:

(A) (pour préparer des composés de formule I dans laquelle $R^2$ n'est pas l'hydrogène) la cyclisation d'un amide d'acide carboxylique de formule générale II

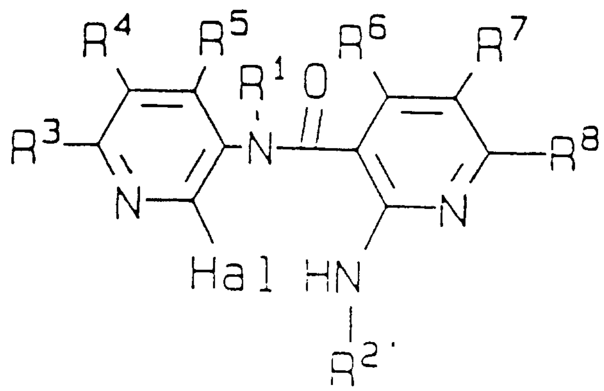

(II)

(dans laquelle $R^1$, $R^3$, $R^4$, $R^5$, $R^6$, $R^7$ et $R^8$ sont tels que définis ci-dessus, $R^{2'}$ est tel que défini pour $R^2$ à l'exception de l'hydrogène et Hal représente un atome de fluor, de chlore, de brome ou d'iode);

(B) (pour produire des composés de formule I dans laquelle $R^2$ est l'hydrogène) le clivage hydrolytique d'un groupe arylméthyle d'un composé de formule III

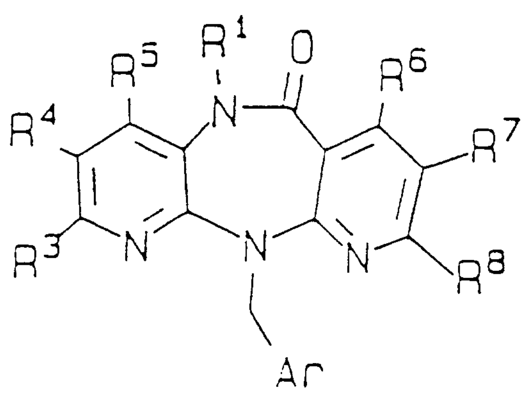

(III)

(dans laquelle $R^1$, $R^3$, $R^4$, $R^5$, $R^6$, $R^7$ et $R^8$ sont tels que définis ci-dessus et Ar représente un groupe aryle);

(C) (pour préparer des composés de formule I dans laquelle $R^1$ n'est pas l'hydrogène) la conversion d'un composé de formule I dans laquelle $R^1$ est l'hydrogène en un composé 5-alcalin ou alcalinoterreux correspondant puis la réaction dudit composé alcalin ou alcalinoterreux avec un composé de formule IV

$R^{1'}X$ (IV)

(dans laquelle $R^{1'}$ est tel que défini ci-dessus pour $R^1$ à l'exception de l'hydrogène et X est le radical d'un ester réactif, un atome d'halogène, un groupe $OSO_2OR^{1'}$, un groupe méthanesulfonyloxy ou éthanesulfonyloxy ou un groupe sulfonyloxy aromatique;

(D) (pour préparer des composés de formule I dans laquelle $R^1$ n'est pas l'hydrogène)

par réaction d'un composé de formule I dans laquelle $R^1$ est l'hydrogène avec un composé de formule IV (tel que défini ci-dessus) en présence d'une amine ou d'un carbonate ou bicarbonate de métal alcalin;

(E) (pour préparer un composé de formule I dans laquelle $R^2$ n'est pas un groupe alcanoyle, hydroxyalkyle ou alcoxycarbonylméthyle)

la conversion d'un composé de formule I dans laquelle $R^2$ est l'hydrogène en un sel métallique correspondant de formule Va ou, lorsque $R^1$ représente l'hydrogène, Vb

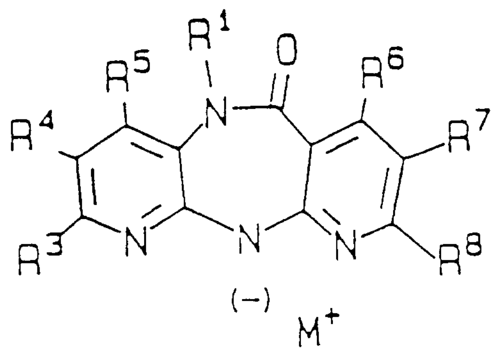

(Va)

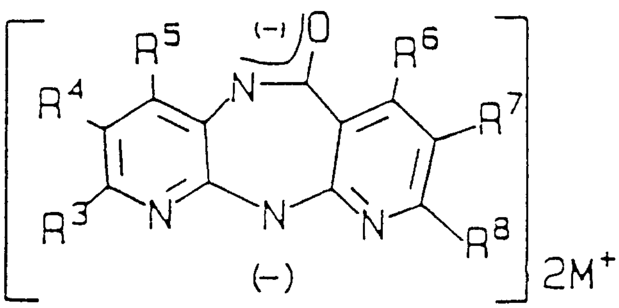

(Vb)

(dans laquelle M représente un métal alcalin comme le lithium, le sodium, le potassium, le rubidium ou le césium, ou bien M représente le groupe $MgHal^+$ dans lequel Hal est le chlore, le brome ou l'iode) puis l'alkylation avec un composé de formule VI

$R^{2''}X$ (VI)

(dans laquelle X est tel que défini ci-dessus) et $R^{2''}$ est tel que défini pour $R^2$ à l'exception de alcanoyle, hydroxyalkyle et alcoxycarbonylméthyle);

(F) (pour préparer un composé de formule I dans laquelle Z est le soufre)

par réaction d'un composé de formule I dans laquelle Z est l'oxygène avec un agent de sulfuration.

7. Utilisation d'un composé de formule I ou de l'un de ses sels d'addition d'acide acceptables du point de vue physiologique selon l'une quelconque des revendications 1 à 4 pour la préparation d'un agent thérapeutique pour conbattre une infection par HIV.

8. Composé de formule I selon l'une quelconque des revendications 1 à 3 ou l'un de ses sels d'addition d'acide acceptables du point de vue physiologique en tant que substance active du point de vue pharmaceutique.